# EUROPEAN PATENT APPLICATION

(11) **EP 4 696 216 A1**
(43) Date of publication of application: **18.02.2026**
(21) Application number: 24194623.5
(22) Date of filing: 14.08.2024
(51) Int. Cl.: A61B 3/00, A61B 3/107, A61B 3/11, A61B 3/117

(54) **A COMPUTER-IMPLEMENTED METHOD FOR OPTO-MECHANICAL EYE MODELLING**

(71) Applicant: Universiteit Antwerpen, 2000 Antwerpen (BE)
(72) Inventor: GHADERI, Hosna, 2020 Antwerp (BE); ROZEMA, Jos J., 2020 Antwerp (BE)
(74) Representative: Ipsilon Belgium

(57) **Abstract**

Example embodiments relate to a computer-implemented method for generating an opto-mechanical model of a camera-type eye from ocular biometry parameters that define the anatomy of the camera-type eye; wherein the ocular biometry parameters comprise at least a pupil size (224) and an iris thickness (223). The computer-implemented method comprises obtaining a predetermined iris shape (220) and a predetermined ciliary body shape (230); anchoring (201) one or more data points (221) of an outer edge of the iris shape (220) to one or more reference points of the posterior corneal surface; transforming (202) the iris shape (220) as to respect the pupil size (224) and the iris thickness (223); anchoring (203) a first portion of the outer edge (250) of the ciliary body shape to a portion of an outer edge (225) of the iris shape; transforming (204) the ciliary body shape (230) such that a second portion of an outer edge (231) of the ciliary body shape coincides with a portion of the inner scleral surface (212); and generating (205) one or more polygons (261, 262) indicative for respective zonular fibres, wherein the respective one or more polygons are defined by at least two data points located on the crystalline lens surface (240) and at least two data points located on an inner edge (232) of the ciliary body shape.

## Description

### Field of the Invention

The present invention generally relates to opto-mechanical modelling of camera-type eyes.

### Background of the Invention

An opto-mechanical model of an eye refers to a computer model or simulation that allows analysing changes in the optical properties of the eye in response to mechanical stimuli, e.g. changes in intraocular pressure, IOP, deformation of ocular tissues, or surgical interventions. This allows exploring the relationship between tissue's biomechanical properties and optical outcomes, i.e. vision. As such, opto-mechanical eye models can be used in the development of surgical techniques, ophthalmic surgery planning, disease modelling, drug impact studies, prosthetic design, and implant design.

Most opto-mechanical models are developed for a particular application and are based on averaged biometric data that does not consider the interindividual differences between eyes, making them less effective for predicting or diagnosing conditions on an individual level. It remains a challenge to efficiently generate an opto-mechanical model for a specific individual's eye as it typically requires extensive ocular biometry measurements and time-consuming manual development by a qualified expert. Moreover, the inaccessibility of some ocular biometry measurement techniques to determine the exact shape and dimensions of some ocular components such as, for example, the iris, the ciliary body, and the zonular fibres in individuals remains a bar to efficiently generate individualized opto-mechanical eye models.

It is a further challenge that most ocular components are interconnected, i.e. a mechanical stimulus applied to a first ocular component typically influences several other interconnected ocular components. As such, a mechanical stimulus applied to one ocular component can affect multiple optical properties of the eye defined by the interconnected ocular components. These effects can be underestimated or even remain completely unnoticed if the interconnectivity of the individual's ocular components is not modelled properly.

### Summary of the Invention

It is an object of the present invention, amongst others, to solve or alleviate above identified problems and challenges by improving the effectiveness of opto-mechanical eye models to predict or diagnose conditions on an individual level by providing a computer-implemented method for generating an opto-mechanical model of a camera-type eye that considers the interindividual differences between eyes and the interconnectivity of ocular components in an efficient way.

According to a first aspect, this object is achieved by a computer-implemented method for generating an opto-mechanical model of a camera-type eye from ocular biometry parameters that define the anatomy of the camera-type eye; wherein the ocular biometry parameters comprise at least a pupil size and an iris thickness; the computer-implemented method comprising:
- obtaining a posterior corneal surface, an inner scleral surface, and a crystalline lens surface;
- obtaining a predetermined iris shape indicative for a generic shape of the iris in a general population, and a predetermined ciliary body shape indicative for a generic shape of the ciliary body in the general population;
- anchoring one or more data points of an outer edge of the iris shape to one or more reference points of the posterior corneal surface;
- transforming the iris shape as to respect the pupil size and the iris thickness;
- anchoring a first portion of the outer edge of the ciliary body shape to a portion of an outer edge of the iris shape;
- transforming the ciliary body shape such that a second portion of an outer edge of the ciliary body shape coincides with a portion of the inner scleral surface; and
- generating one or more polygons indicative for respective zonular fibres, wherein the respective one or more polygons are defined by at least two data points located on the crystalline lens surface and at least two data points located on an inner edge of the ciliary body shape.

A camera-type eye refers to a type of ocular structure comprising a single, adjustable lens that focuses light onto a photosensitive surface, e.g. a retina. A camera-type eye typically comprises a plurality of interconnected ocular components such as, for example, a crystalline lens, a pupil, a retina, a cornea, an iris, a sclera, a ciliary body, and zonular fibres. Camera-type eyes are present in humans; other vertebrate species such as, for example, mice and chicken; and in some invertebrate species such as, for example, cephalopods.

Ocular biometry parameters refers to quantitative measurements of the eye's anatomical and/or optical characteristics. Common ocular biometry parameters include for example, amongst others, axial length, corneal curvature, corneal curvature maps, corneal refractive index, corneal diameter, anterior chamber depth, crystalline lens thickness, crystalline lens curvature, crystalline lens curvature maps, crystalline lens diameter, crystalline lens gradient refractive index, pupil diameter, pupil size, corneal elevation, corneal thickness, vitreous depth, and retinal curvature. Ocular biometry parameters may be measured using specialised instruments, e.g. ultrasound biometry, optical coherence tomography, OCT, and partial coherence interferometry, PCI. Alternatively, some ocular biometry parameters may be obtained from a generative eye model configured to generate one or more sets of ocular biometry parameters indicative for the anatomy of respective `virtual' eyes, e.g. as disclosed in 'Rozema JJ, et al., SyntEyes: a higher order statistical eye model for healthy eyes. Invest Ophthalmol Vis Sci. 2016;57:683-691. DOI:10.1167/iovs.15-18067'. A 'virtual eye' may be a representation of an imaginary eye that is anatomically plausible and similar to the eyes in the general population.

Obtaining the posterior corneal surface, the inner scleral surface, and the crystalline lens surface may be achieved based on one or more ocular biometry parameters, e.g. by measurement and/or calculations. At least the pupil size and the iris thickness are provided as input for the computer-implemented method. The pupil size, sometimes also referred to as pupil diameter, refers to a measurement indicative of the pupil's width. The iris thickness refers to a measurement indicative of the width of the iris.

The steps of the computer-implemented method may be performed in a two-dimensional plane, e.g. a hemi-sagittal cross-sectional plane. In other words, ocular components may be modelled or represented as two-dimensional elements in half of a cross-section of the eye along the sagittal plane. This half may, for example, be an upper half or a lower half of the eye. The obtained posterior corneal surface, the inner scleral surface, and the crystalline lens surface may therefore represent a cross section of the posterior corneal surface, inner scleral surface, and the crystalline lens surface, respectively. Performing the computer-implemented method in a two-dimensional plane has the advantage that it has reduced computational complexity.

Alternatively, the computer-implemented method may be performed in a three-dimensional space. In other words, ocular components may be modelled or represented as three-dimensional elements in three-dimensional space. This has the advantage that irregular shapes of ocular components can be modelled more accurately.

Further, a predetermined iris shape and a predetermined ciliary body shape are obtained representing a generic shape of the iris and ciliary body in the general population, respectively. The general population may refer to a general population of the species for which the camera-type eye is modelled, e.g. it may refer to a general population of mice when modelling the eyes of mice and may refer to a general population of humans when modelling the eyes of humans. The predetermined iris shape and the predetermined ciliary body shape may, for example, be obtained by fitting a two-dimensional or three-dimensional shape to a set of measurements of the iris and ciliary body in the general population.

The predetermined iris shape is then anchored to the posterior corneal surface and transformed such that the pupil size and iris thickness, which are provided as input parameters, are respected. By anchoring the iris shape to the posterior corneal surface, the position of the iris shape changes if the position or shape of the corneal surface changes, e.g. due to a mechanical stimulus. By transforming the iris shape according to the pupil size and iris thickness, the iris shape further maintains a truthful shape even in the presence of mechanical stimuli. This allows creating an adaptive individualized eye model, as the generic iris shape is adjusted to the pupil size, iris thickness, and the posterior corneal surface.

A first portion of the outer edge of the predetermined ciliary body shape, i.e. its periphery, is in turn anchored to the anchored iris shape. The ciliary body shape is further transformed such that a second portion of an outer edge of the ciliary body shape coincides with a portion of the inner scleral surface. Thus, an outer edge of the ciliary body shape is transformed or reshaped to coincide with a portion of the inner scleral surface while respecting the anchored common edge between the iris shape and ciliary body shape. In doing so, the position and shape of the ciliary body changes directly when the position or shape of the iris changes and/or when the position or shape of the inner scleral surface changes. Additionally, the position and shape of the ciliary body also changes indirectly when the position or shape of the posterior corneal surface changes as it is anchored to the iris shape which, in turn, is anchored to the posterior corneal surface. Transforming the ciliary body shape further allows creating an adaptive individualized eye model as the generic ciliary body shape can be adapted to an inner scleral surface curvature specific to a certain subject or patient.

By modelling one or more zonular fibres as polygons connecting the inner edge of the ciliary body shape with the crystalline lens surface, the generated opto-mechanical eye model can account for the interconnectivity between the ciliary body and the crystalline lens. This allows determining how mechanical or physiological changes of the ciliary body affect the shape of the crystalline lens, which is critical for assessing the optical properties of the eye. Moreover, the interconnectivity of the modelled ocular components allows assessing the effect of mechanically stimulating one or more ocular components on the optical properties or optics of the eye.

It is thus an advantage that the position and shape of multiple modelled ocular components are interdependent, resulting in a more adaptive and improved opto-mechanical model of the eye. This has the advantage that an opto-mechanical model generated according to the computer-implemented method can be more effective at predicting the optical outcomes of mechanically stimulating the eye's tissue.

The computer-implemented method further allows reducing the time and effort to generate individualized opto-mechanical anatomic models of the eye, as it uses predetermined generic shapes to model ocular components that are adjusted according to only a few of an individual's ocular biometry parameters. This has the advantage that an opto-mechanical model generated according to the computer-implemented method can be more effective at predicting ophthalmologic conditions and predicting the effect of mechanical stimuli on the eye's optics on an individual level.

It is a further advantage that the computer-implemented method can easily be automated. It is a further advantage that the computer-implemented method can make complex modelling of eyes more accessible to a wider range of professionals and researches, as it merely requires a limited number of ocular biometry parameters as input without further manual interventions by a qualified expert.

According to an example embodiment, anchoring the one or more data points of the outer edge of the iris shape to the one or more reference points of the posterior corneal surface may comprise positioning the iris shape such that the one or more data points coincide with one or more reference points of the posterior corneal surface.

In a two-dimensional plane, a single data point of the outer edge of the iris shape may be anchored to a single reference point of the posterior corneal surface. In a three-dimensional space, a plurality of data points of the outer edge of the iris shape may be anchored to a plurality of reference points of the posterior corneal surface, e.g. a curve or surface of the iris shape may be anchored to a curve or surface of the posterior corneal surface.

According to an example embodiment, the one or more anchored data points may be the data points on the outer edge of the iris shape that are anatomically located at the greatest distance from a rotational axis along a longitudinal axis and anatomically located at the smallest distance to an apex of the posterior corneal surface along the rotational axis.

The rotational axis may be the axis that allows generating a three-dimensional representation of the eye by rotating two-dimensional cross-sections of the ocular components around it. The rotational axis may, for example, pass through the centre of the cornea and the centre of the crystalline lens. In other words, the rotational axis may substantially coincide with the optical axis in some cases. The longitudinal axis may be substantially perpendicular to the rotational axis. The one or more anchored data points of the iris shape may thus be the data points belonging to the iris shape that are located furthest away from the rotational axis and simultaneously closest to the apex of the posterior corneal surface when the iris shape is positioned anatomically correctly within a two-dimensional plane or three-dimensional space. The one or more anchored data points may, for example, be located near the scleral spur.

According to an example embodiment, the one or more reference points of the posterior corneal surface may be data points where the posterior corneal surface and the inner scleral surface intersect.

In other words, the one or more reference points where the one or more data points of the iris shape are anchored to may be located substantially in the vicinity of the limbus of the eye, i.e. the border or junction between the cornea and the sclera.

According to an example embodiment, anchoring the first portion of the outer edge of the ciliary body shape comprises positioning the ciliary body shape such that the first portion of the outer edge coincides with the portion of the outer edge of the iris shape.

According to an example embodiment, the first portion of the outer edge of the ciliary body shape and the portion of the outer edge of the iris shape coincide with the one or more reference points of the posterior corneal surface.

According to an example embodiment, the portion of the inner scleral surface according to which the ciliary body shape is transformed may be the portion of the inner scleral surface adjacent to the posterior corneal surface.

In other words, transforming the ciliary body shape may be performed such that the second portion of the outer edge of the ciliary body shape coincides with the portion of the inner scleral surface adjacent to the posterior corneal surface. An end point of this portion may be the data point where the posterior corneal surface and the inner scleral surface intersect, i.e. in the vicinity of the limbus of the eye.

According to an example embodiment, the ocular biometry parameters further comprise a length of a ciliary body along a rotational axis of the eye; and wherein transforming the ciliary body shape further comprises transforming the ciliary body shape as to respect the length of the ciliary body along the rotational axis.

Thus, in addition to transforming or reshaping the generic ciliary body shape according to the inner scleral surface, the generic ciliary body shape may further be transformed or reshaped such that its length corresponds to an input parameter indicative for the length of the ciliary body. This serves as an additional constraint for transforming the generic ciliary body according to an individual's ocular biometry, thereby resulting in an individualized ciliary body shape that is anatomically more correct.

According to an example embodiment, transforming the ciliary body shape further comprises transforming the ciliary body shape as to align a ciliary body apex with a crystalline lens equator along a longitudinal axis.

The ciliary body apex may be located along the inner edge of the ciliary body shape. The ciliary body apex may be a protruding or bulging portion of the inner edge of the ciliary body in the direction of the crystalline lens surface. The protruding or bulging portion may be relatively narrow, extend almost up to the crystalline lens surface, and taper towards the iris. The crystalline lens equator may refer to a point on the peripheral edge of the crystalline lens surface where the lens is relatively thin and relatively close to where the zonular fibres connect to the lens. Aligning the ciliary body apex with the crystalline lens equator serves as an additional constraint for transforming the generic ciliary body according to an individual's ocular biometry, thereby resulting in an individualized ciliary body shape that is anatomically more correct.

According to an example embodiment, the at least two data points of the respective one or more polygons located on the crystalline lens surface may be located on the anterior lens surface or posterior lens surface at respective predetermined distances from a crystalline lens equator along a longitudinal axis.

In other words, the data points of the respective polygons are located at predetermined distances from the crystalline lens equator along an axis perpendicular to the rotational axis. These data points are located somewhere along the crystalline lens surface, i.e. the outer edge of the crystalline lens. This may be the posterior lens surface or the anterior lens surface. For example, the data points of a first polygon may be located on the anterior lens surface, the data points of a second and a third polygon may be located substantially at the crystalline lens equator, and the data points of a fourth and fifth polygon may be located on the posterior crystalline lens surface. These data points of the respective polygons may also be referred to as the anchoring points of the respective polygons.

According to an example embodiment, the at least two data points of the respective one or more polygons located on the inner edge of the ciliary body shape are located at respective predetermined distances from a ciliary body apex along the rotational axis.

For example, the data points of a first polygon may be located anteriorly to the ciliary body apex, the data points of a second polygon may be located substantially at the ciliary body apex, the data points of a third polygon and fourth polygon may be located at the posterior crystalline lens surface, and the data point of a fifth polygon may be located at around the most posterior point of the ciliary body shape.

According to an example embodiment, the computer-implemented method may be performed in a hemi-sagittal cross-sectional plane and the computer-implemented method may further comprise rotating the shapes (220, 230) and surfaces (211, 212, 240) within the hemi-sagittal cross-sectional plane (210) around the rotational axis (213) to generate a three-dimensional, 3D, model of the eye.

The shapes refer to the iris shape, the ciliary body shape, and the one or more polygons indicative for the zonular fibres. The surfaces refer to the posterior corneal surface, the inner scleral surface, and the crystalline lens surface. By rotating the shapes and surfaces around the rotational axis, respective volumes are generated indicative for respective ocular components. This may result in a substantially complete three-dimensional opto-mechanical eye model that allows improved investigation of the effect of mechanical stimuli on the optics of the eye due to the interconnectivity of the ocular components.

According to a second aspect, the object is achieved by a data processing system configured to perform the computer-implemented method according to the first aspect.

According to a third aspect, the object is achieved by a computer program comprising instructions which, when the computer program is executed by a computer, cause the computer to perform the computer-implemented method according to the first aspect.

According to a fourth aspect, the object is achieved by a computer-readable medium comprising instructions which, when executed by a computer, cause the computer to perform the computer-implemented method according to the first aspect.

### Brief Description of the Drawings

Fig. 1 shows a schematic example of a sagittal cross-sectional anatomy of a camera-type eye;
Fig. 2 shows steps of a computer-implemented method for generating an opto-mechanical model of a camera-type eye from ocular biometry parameters, according to example embodiments;
Fig. 3 shows the anchoring of a data point of an outer edge of the iris shape to a reference point of the posterior corneal surface, according to example embodiments;
Fig. 4 shows the anchoring of the ciliary body shape to the iris shape and transforming the ciliary body shape to coincide with the inner scleral surface, according to example embodiments;
Fig. 5 shows the generating of one or more polygons indicative for respective zonular fibres, according to example embodiments; and
Fig. 6 shows an example embodiment of a suitable computing system for performing steps according to example aspects of the invention.

### Detailed Description of Embodiment(s)

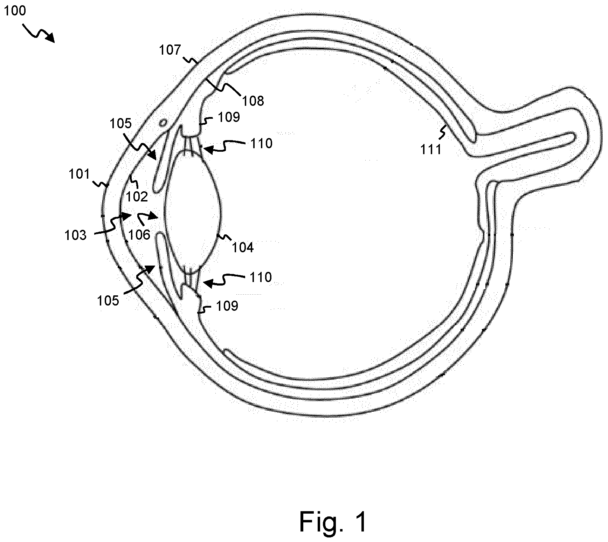

Fig. 1 shows a schematic example of the sagittal cross-sectional anatomy of a camera-type eye 100. A camera-type eye refers to a type of ocular structure comprising a single, adjustable lens 104 that focuses light onto a photosensitive surface, e.g. the retina 111. A camera-type eye typically comprises a plurality of interconnected ocular components such as, for example, a crystalline lens 104, a pupil 106, a retina 111, a cornea 101, 102, an iris 105, a sclera 107, 108, a ciliary body 109, and zonular fibres 110. Camera-type eyes are present in humans and other vertebrate species, e.g. mice and chickens. Camera-type eyes may also be present in some invertebrate species such as cephalopods, e.g. octopuses, squids, and cuttlefish.

The cornea 101, 102 is a clear, dome-shaped outermost layer of the eye that covers the iris 105 and the pupil 106. It is the eye's primary refractive surface, bending light rays as they enter the eye to help focus them onto the retina 111. The cornea comprises an anterior cornea 101 and a posterior cornea 102. The sclera 107, 108 is the white, opaque part of the eye that surrounds the cornea 101, 102 and covers most of the eye's surface, providing protection and structural support. It acts as a protective outer layer for the eye and maintains the shape of the eyeball. It further serves as an attachment point for the eye muscles that control eye movement. The sclera comprises an outer sclera 107 and an inner sclera 108.

The pupil 106 is a circular opening at the centre of the iris 105. The pupil 106 regulates the amount of light entering the eye by changing size in response to light intensity. The size of the pupil 106 is controlled by the iris 105. This is achieved through the contraction and relaxation of the iris muscles, i.e. the sphincter and dilator muscles, thereby regulating the amount of light that enters the eye. The iris 105 and pupil 106 are located anterior to the crystalline lens 104. The crystalline lens 104 is a transparent, biconvex structure that is flexible and adjustable. The crystalline lens 104 focuses light onto the retina 111 by adjusting its shape through the process of accommodation. This allows the eye to focus on objects at various distances. The shape of the crystalline lens 104 is adjusted by contracting and relaxing the muscles within the ciliary body 109, which is connected to the crystalline lens 104 by means of zonular fibres 110.

The interconnectivity of these ocular components has the result that a change in position or shape of one ocular component can affect the position or shape of a plurality of other, interconnected ocular components, e.g. a change in position or shape of the ciliary body 109 may affect the shape and/or position of the zonular fibres 110, the crystalline lens 104, the iris 105, and/or the cornea 101, 102. As the shape and position of the latter ocular components 101, 102, 104, 105 mainly determine the optical properties of a camera-type eye 100, the interconnectivity of the ocular components poses a challenge to effectively determine the optical properties of an eye in the presence of mechanical stimuli, e.g. muscle contractions, muscle relaxations, changes in intraocular pressure, or external manipulations such as LASIK or surgical incisions.

It is a further challenge to determine these optical properties in the presence of mechanical stimuli for eyes of specific individuals or subjects. Most opto-mechanical models have been developed for a particular application and are based on averaged data that does not consider the interindividual differences between eyes, making them less effective for predicting or diagnosing conditions on an individual level. As such, generating an opto-mechanical model for a specific individual's eye typically requires time-consuming manual development by a technical expert. Moreover, the inaccessibility of some measurement techniques to determine the exact shape and dimensions of some ocular components such as, for example, the iris, the ciliary body, and the zonular fibres, in individuals remains a challenge to efficiently generate individualized opto-mechanical eye models.

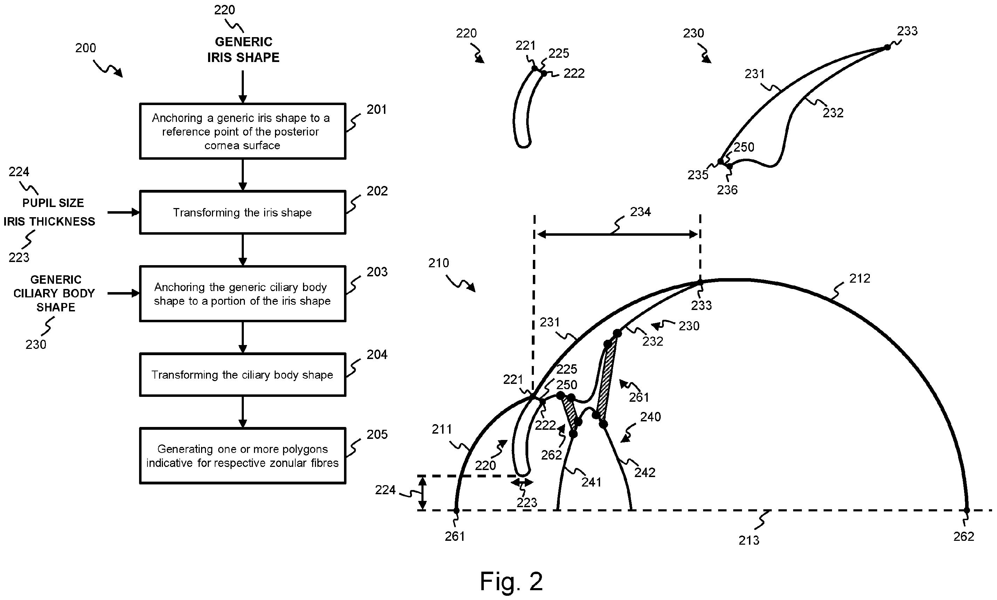

Fig. 2 shows steps 200 of a computer-implemented method for generating an opto-mechanical model of a camera-type eye that at least partially solves above-mentioned challenges, according to example embodiments.

The steps 200 of the computer-implemented method may be performed in a two-dimensional plane, e.g. in a hemi-sagittal cross-sectional plane 210. In other words, ocular components may be modelled or represented as two-dimensional elements in half of a cross-section of the eye along the sagittal plane, i.e. a vertical slice from the anterior to the posterior of the eye. The sagittal plane refers to an anatomical term used to describe a vertical plane that divides a body into left and right portions. The hemi-sagittal cross-section may, for example, be an upper half of the sagittal cross-section of the eye or a lower half of the sagittal cross-section of the eye. Fig. 2 shows an example 210 of an upper hemi-sagittal cross-section of an eye.

It will be apparent that the computer-implemented method may also be performed in a three-dimensional space. In other words, ocular components may be modelled or represented as three-dimensional elements in three-dimensional space, e.g. curves, surfaces, or volumes. For clarity, Fig. 2 - 5 only show two-dimensional examples. In particular, Fig. 2 - 5 illustrate how the computer-implemented method may be performed in a hemi-sagittal cross-sectional plane.

In a first step (not shown in Fig. 2), a hemi-sagittal cross-section of some ocular components is obtained. These ocular components include a posterior corneal surface 211 indicative for a hemi-sagittal cross-section of the posterior cornea 102, an inner scleral surface 212 indicative for a hemi-sagittal cross-section of the inner sclera 108, and a crystalline lens surface 240 indicative for a hemi-sagittal cross-section of the crystalline lens 104. These surfaces 211, 212, 240 may thus be provided as an input to the computer-implemented method 200. Alternatively, these surfaces 211, 212, 240 may be obtained from one or more ocular biometry parameters.

Ocular biometry parameters refer to quantitative measurements of the eye's anatomical and/or optical characteristics. Common ocular biometry parameters include for example, amongst others, axial length, corneal curvature, anterior chamber depth, crystalline lens thickness, crystalline lens curvature, pupil diameter, pupil size, corneal elevation, intraocular distance, corneal thickness, and vitreous depth. Ocular biometry parameters may be measured using specialised instruments, e.g. ultrasound biometry, optical coherence tomography, OCT, and partial coherence interferometry, PCI. Alternatively, some ocular biometry parameters may be obtained from a generative eye model configured to generate one or more sets of ocular biometry parameters indicative for the anatomy of respective `virtual' eyes, e.g. as disclosed in 'Rozema JJ, et al., SyntEyes: a higher order statistical eye model for healthy eyes. Invest Ophthalmol Vis Sci. 2016;57:683-691. DOI:10.1167/iovs.15-18067'. A 'virtual eye' may be a representation of an imaginary eye that is anatomically plausible and similar to the eyes in the general population, i.e. statistically indistinguishable from an eye in the general population.

Thus, surfaces 211, 212, 240 may be obtained from one or more of these ocular biometry parameters. For example, the posterior corneal surface 211 may be obtained by fitting a surface or curve to measurements of the posterior corneal elevations. The inner scleral surface 212 may be obtained by fitting a portion of a circle connecting an end point of the posterior corneal surface 211 with a point 262 located on the rotational axis 213. This point 262 may be located at a distance equal to the axial length from the most anterior point 261 of the posterior corneal surface 211. Alternatively, one or more of the surfaces 212, 240 may be obtained directly from magnetic resonance imaging, MRI, images.

The computer-implemented method further receives at least two ocular biometry parameters as an input: a pupil size 224 and an iris thickness 223. The pupil size 224, sometimes also referred to as pupil diameter, refers to a measurement indicative of the pupil's 106 width. The iris thickness 223 refers to a measurement indicative of the width of the iris 105.

The computer-implemented method further obtains a generic iris shape 220 and a generic ciliary body shape 230 as input. These generic shapes 220, 230 are predetermined. These generic shapes 220, 230 represent a generic shape of the iris 220 and a generic shape of the ciliary body 230 in the general population, e.g. generic hemi-sagittal cross-sectional shapes. The general population may refer to a general population of the species for which the camera-type eye is modelled, e.g. it may refer to a general population of mice when modelling the eyes of mice and may refer to a general population of humans when modelling the eyes of humans. The predetermined iris shape 220 and the predetermined ciliary body shape 230 may, for example, be obtained by fitting a two-dimensional or three-dimensional shape to a set of average ocular biometry measurements of the iris and ciliary body in the general population. The generic iris shape 220 and the generic ciliary body shape 230 may be defined by their outer boundary, also referred to as periphery, edge, or outer limit.

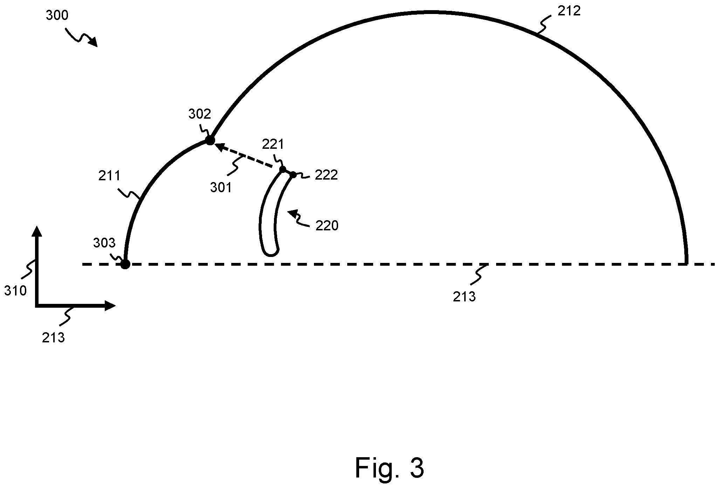

In a next step 201, a data point 221 of an outer edge of the predetermined generic iris shape 220 is anchored to a reference point of the posterior corneal surface 211. This is further illustrated in Fig. 3, which shows an example of the anchoring step 201 in a hemi-sagittal cross-sectional plane, according to embodiments.

Anchoring the generic iris shape 220 to the posterior corneal surface 211 may be achieved by positioning 301 the generic iris shape 220 within the hemi-sagittal cross-sectional plane 300 such that the position of the data point 221 within the hemi-sagittal cross-sectional plane 300 is fixed relative to the reference point 302. Preferably, the iris shape 220 is positioned such that the anchored data point 221 coincides with the reference point 302 of the posterior corneal surface 211, i.e. such that the anchored data point 221 and the reference point 302 have the same coordinates within the hemi-sagittal cross-sectional plane 300. It will be apparent that, when the computer-implemented method is performed in a three-dimensional space, a plurality of data points of the iris shape 220 indicative for a closed curve or surface may be anchored to a plurality of data points of the posterior corneal surface.

The anchored data point 221 is an integral part of the boundary or periphery of the generic iris shape 220, i.e. the anchored data point 221 is a defining point that makes up the outer edge of the generic iris shape 220. The anchored data point 221 may be the data point of the iris shape 220 that is anatomically located at the greatest distance from the rotational axis 213 along a longitudinal axis 310 and anatomically located at the smallest distance to an apex 303 of the posterior corneal surface 211 along the rotational axis 213. The anchored data point 221 of the iris shape 220 may thus be the data point of the iris shape 220 that is located furthest away from the rotational axis 213 and simultaneously located closest to the apex 303 of the posterior corneal surface 211 when the iris shape 220 is positioned anatomically correct within the hemi-sagittal cross-sectional plane 300. The anchored data point 221 may, for example, be located near the scleral spur. It will be apparent that the longitudinal axis 310 may be substantially perpendicular to the rotational axis 213. The rotational axis 213 may be the axis that allows generating a three-dimensional representation of the eye by rotating the two-dimensional cross-sections 211, 212, 220, 230, 240 of the ocular components around it.

The reference point 302, i.e. the data point to which the anchored data point 221 is anchored, may be a data point where the posterior corneal surface 211 and the inner scleral surface 212 intersect. In other words, the reference point 302 may be located substantially in the vicinity of the limbus of the eye, i.e. the border or junction between the cornea 101, 102 and the sclera 107, 108.

Returning to Fig. 2, a next step 202 comprises transforming the predetermined generic iris shape 220 as to respect the pupil size 224 and the iris thickness 223 obtained as inputs. Transforming may, for example, include scaling, rotating, shearing, translating, and reshaping the generic iris shape 220. It will be apparent that the generic iris shape 220 is transformed relative to the anchored data point 221, i.e. without changing the location or position of the anchored data point 221 within the hemi-sagittal cross-sectional plane 210. For example, scaling the generic iris shape 220 may include growing or shrinking the generic iris shape 220 while the anchored data point 221 remains located in the same position, i.e. relative to the reference point 302 on the posterior corneal surface 211.

By anchoring 201 the iris shape 220 to the posterior corneal surface 211, the position of the iris shape 220 changes if the position or shape of the corneal surface 211 changes, e.g. due to a mechanical stimulus. By transforming 202 the iris shape 220 according to the pupil size 224 and iris thickness 223, the iris shape further maintains a truthful shape even in the presence of mechanical stimuli. This allows creating an individualized eye model, as the generic iris shape 220 can be adjusted to the pupil size 224, iris thickness 223, and the posterior corneal surface 211 specific to a certain subject or patient. Additionally, it allows creating an adaptive eye model, as the iris shape 220 can adapt to changes in position, shape, or dimensions of other, interconnected ocular components, e.g. an increase in pupil size or a change in intraocular pressure.

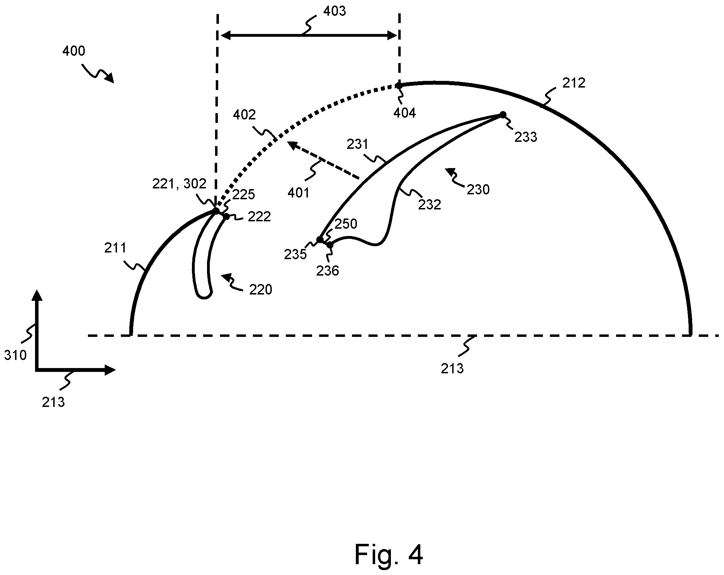

In a following step 203, a first portion 250 of the outer edge of the predetermined generic ciliary body shape 230 is anchored to a portion of the outer edge of the anchored iris shape 220. This is further illustrated in Fig. 4, which shows an example of the anchoring step 203 in a hemi-sagittal cross-sectional plane, according to embodiments.

Anchoring 203 the first portion 250 of the outer edge of the predetermined generic ciliary body shape 230 may be achieved by positioning 401 the generic ciliary body shape 230 within the hemi-sagittal cross-sectional plane 400 such that the position of the first portion 250 of the outer edge is fixed relative to the portion 225 of the iris shape 220. Preferably, the ciliary body shape 230 is positioned such that the first portion 250 of the outer edge of the ciliary body shape 230 coincides with the portion 225 of the iris shape 250, thereby establishing a common edge between the iris shape 220 and the ciliary body shape 230. This can, for example, be achieved by positioning the ciliary body shape 230 such that data point 235 and data point 236 of the ciliary body shape 230 coincide with data point 221 and data point 222 of the iris shape 220, respectively. In doing so, the data point 235 of the ciliary body shape is also indirectly anchored to the reference point 302 on the posterior corneal surface 211 and, thus, the ciliary body shape 230 is indirectly anchored to the posterior corneal surface 211.

Returning to Fig. 2, a next step 204 comprises transforming the generic ciliary body shape 230 such that a second portion 231 of the outer edge of the ciliary body shape 230 coincides with a portion of the inner scleral surface 212. Preferably, this is the portion of the inner scleral surface adjacent to the posterior corneal surface 211, as illustrated by portion 402 in Fig. 4. In other words, transforming the ciliary body shape 230 may be performed such that the second portion 231 of the outer edge of the ciliary body shape 230 coincides with portion 402 of the inner scleral surface 211 adjacent to the posterior corneal surface 211. An end point of this portion 402 may be reference point 302, i.e. the data point where the posterior corneal surface 211 and the inner scleral surface 212 intersect near the limbus. Transforming the generic ciliary body shape 230 may, for example, include scaling, rotating, shearing, translating, and reshaping the ciliary body shape 230. It will be apparent that the ciliary body shape 230 is transformed relative to the anchored edge portions 225, 250, i.e. without changing the location or position of the anchored edge portion 250 within the hemi-sagittal cross-sectional plane 210.

In doing so, the position and shape of the ciliary body 230 changes directly when the position or shape of the iris changes 220 and/or when the position or shape of the inner scleral surface 212 changes. Additionally, the position and shape of the ciliary body 230 also changes indirectly when the position or shape of the posterior corneal surface 211 changes as it is anchored to the iris shape 220 which, in turn, is anchored to the posterior corneal surface 211. Transforming 204 the ciliary body shape 230 further allows creating an adaptive individualized eye model as the generic ciliary body shape 230 can be adapted to an inner scleral surface curvature specific to a certain subject or patient.

Transforming the ciliary body shape 230 may further comprise transforming the ciliary body shape as to respect a length 403 of the ciliary body along the rotational axis 213. To this end, the computer-implemented method may further receive, as an additional ocular biometry parameter input, the length 403 of a ciliary body. This may, for example, be a desired length of the ciliary body or a measurement of the ciliary body length in a specific subject or patient. This transforming can, for example, be achieved by reshaping or scaling the ciliary body shape 230 such that data point 233 coincides with data point 404 on the inner scleral surface 212.

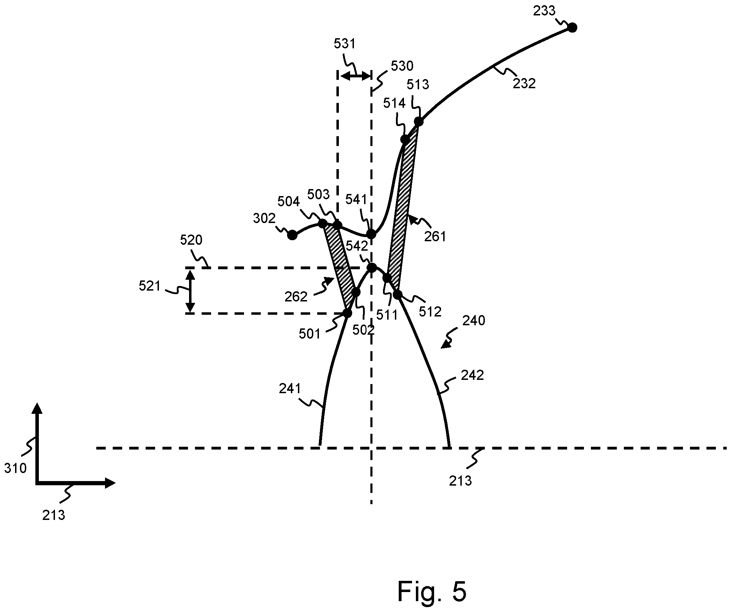

Transforming the ciliary body shape may further comprise transforming the ciliary body shape as to align a ciliary body apex 541 with a crystalline lens equator 542. This is illustrated in Fig. 5. The ciliary body apex 541 refers to a protruding or bulging portion of the inner edge 232 of the ciliary body 230 in the direction of the crystalline lens surface 240. The protruding or bulging portion of the inner edge 232 may be relatively narrow, extend almost up to the crystalline lens surface 240, and taper towards the iris 220. It will be apparent that the inner edge 232 refers to the edge of the ciliary body shape closest to the rotational axis 213 when the ciliary body shape 230 is positioned anatomically correct, i.e. the edge closest to the centre of the eye. The crystalline lens equator 542 may refer to a point on the peripheral edge of the crystalline lens surface 240 where the crystalline lens is thinnest and meets the zonular fibres that hold the lens in place. Aligning the ciliary body apex 541 with the crystalline lens equator 542 serves as an additional constraint for transforming the generic ciliary body 230 according to an individual's ocular biometry, thereby resulting in an individualized ciliary body shape that is anatomically more correct.

Returning to Fig. 2, a next step 205 of the computer-implemented method comprises generating one or more polygons 261, 262 indicative for respective zonular fibres 110. The respective polygons 261, 262 are defined by at least two data points located on the crystalline lens surface 240 and at least two data points located on the inner edge 232 of the ciliary body shape 230. These at least two data points on the inner edge 232 and on the crystalline lens surface 240 may preferably be the vertices of the respective polygons. In other words, one or more zonular fibres are modelled as polygons connecting the inner edge 232 of the ciliary body shape with the crystalline lens surface 240. In doing so, the generated opto-mechanical eye model can account for the interconnectivity between the ciliary body 109 and the crystalline lens 104 of an eye. This allows determining how mechanical or physiological changes of the ciliary body 109 affect the shape of the crystalline lens 104, which is critical for assessing the optical properties of the eye. It will be apparent that the shape of the respective polygons 261, 262 may be adjustable but that the position of their data points remains fixed on the inner edge 232 of the ciliary body shape and the crystalline lens surface 240, i.e. the at least two data points on the inner edge and the at least two data points on the crystalline lens surface may act as anchoring points.

Fig. 5 shows a detailed example of the respective polygons 261, 262 and their data points 501 - 504 and 511 - 514 in a hemi-sagittal cross-sectional plane, according to embodiments. The data points 501, 502, 511, 512 located on the crystalline lens surface 240 may be located on the anterior lens surface 241 or the posterior lens surface 242. Preferably, these data points 501, 502, 511, 512 may be located at respective predetermined distances from the crystalline lens equator 542 along the longitudinal axis 310. For example, data point 501 is located on the anterior lens surface 241 at predetermined distance 521 from the crystalline lens equator 542.

It will further be apparent that Fig. 5 shows a simplified schematic representation of only two polygons 261, 262 but that there may be more or fewer polygons. According to an example embodiment, five polygons indicative for five respective zonular fibres may be generated. At least two data points of the first polygon may then, for example, be located on the anterior lens surface 241 at a predetermined distance of around 0.5 mm from the crystalline lens equator 542. At least two data points of the second polygon may be located substantially at the crystalline lens equator 542. At least two data points of the third polygon may be located on the posterior crystalline lens surface 242 at a predetermined distance of around 0.5 mm from the crystalline lens equator 542. At least two data points of the fourth polygon may be located on the posterior crystalline lens surface 242 at a predetermined distance of around 0.7 mm from the crystalline lens equator 542. At least two data points of the fifth polygon may also be located substantially at the crystalline lens equator 542, similar to the second polygon.

It will be apparent that the at least two data points of the respective polygons are separated by a distance along the longitudinal axis 310 depending on the width of the respective polygons, i.e. the thickness of the modelled zonular fibres. For example, a first of the at least two data points of the fourth polygon may be located at 0.695 mm from the crystalline lens equator 542, and a second of the at least two data points of the fourth polygon may be located at 0.705 mm from the crystalline lens equator 542. This may, for example, result in a polygon having a width of around 10 µm depending on the curvature of the crystalline lens surface 240.

The data points 503, 504, 513, 514 located on the inner edge 232 of the ciliary body shape may further be located at respective predetermined distances from the ciliary body apex 541 along the rotational axis 213. For example, data point 503 is located on the inner edge 232 at predetermined distance 531 from the ciliary body apex 541. It will be apparent that Fig. 5 shows a simplified schematic representation of the polygons 261, 262 and that the data points 503, 504, 514, 513 may be located substantially closer to the ciliary body apex 541. In the example embodiment of five polygons above, the at least two data points of the first, second, third, fourth, and fifth polygon may then respectively be located at around 0.1 mm anterior to the ciliary body apex 541; located at around the ciliary body apex 541;; located at around 0.1 mm posterior to the ciliary body apex 541; located at around 0.2 mm posterior to the ciliary body apex 541; and located at around the most posterior point of the inner edge, i.e. point 233. Thus in this embodiment, the second and fifth polygon both attach to the lens surface 240 at substantially the same location but attach to different locations on the inner edge 232 of the ciliary body shape.

It will be apparent that these at least two data points of the respective polygons are separated by a distance along the rotational axis 213 depending on the width of the respective polygons, i.e. the thickness of the modelled zonular fibres. For example, a first of the at least two data points of the fourth polygon may be located at 0.195mm from the ciliary body apex 541, and a second of the at least two data points of the fifth polygon may be located at 0.205mm from the ciliary body apex 541. This may, for example, result in a polygon having a width of around 10 µm depending on the curvature of the inner edge 232 of the ciliary body shape.

By performing the steps 200 of the computer-implemented method in a two-dimensional plane, a two-dimensional model of a camera-type eye can efficiently be generated that considers the interindividual differences between eyes and the interconnectivity of ocular components. This two-dimensional model can be used to further generate a complete three-dimensional opto-mechanical model. To this end, the computer-implemented method may further comprise rotating the shapes 220, 230, 261, 262 and surfaces 211, 212, 240 around the rotational axis 213 to generate a three-dimensional opto-mechanical model of the eye. In doing so, a three-dimensional element can be obtained for the respective ocular components 211, 212, 220, 230, 240, 261, 262. This three-dimensional eye model can then be used to create a mesh of the ocular components and assign mechanical properties to the respective mesh elements. In other words, the interconnected two-dimensional model can be used as a basis for finite element analysis. As such, the steps of the computer-implemented method can be used to derive or predict the physical state of an existing real object, i.e. a camera-type eye, from measurements of physical properties, i.e. from ocular biometry parameters. Alternatively, the three-dimensional eye model can be obtained directly by performing the computer-implemented method in a three-dimensional space.

The interconnectivity of the modelled ocular components 211, 212, 220, 230, 240, 261, 262 allows assessing the effect on the optical properties or optics of the eye when one or more ocular components are mechanically stimulated. This allows exploring the relationship between tissue biomechanics and optical outcomes for development of surgical techniques, ophthalmic surgery planning, disease modelling, prosthetic and implant design, and drug impact studies.

It is thus an advantage that the position and shape of the modelled ocular components 211, 212, 220, 230, 240, 261, 262 are interdependent, resulting in a more adaptive and accurate opto-mechanical model of the eye. This has the advantage that an opto-mechanical model generated according to the computer-implemented method can be more effective at predicting the optical outcomes of mechanically stimulating the eye's tissue.

The computer-implemented method further allows reducing the time and effort to generate individualized opto-mechanical anatomic models of the eye, as it uses predetermined generic shapes 220, 230 to model ocular components that are adjusted according to some of an individual's ocular biometry parameters. This has the advantage that an opto-mechanical model generated according to the computer-implemented method can be more effective at predicting ophthalmologic conditions and predicting the effect of mechanical stimuli on the eye's optics on an individual level.

It is a further advantage that the computer-implemented method can easily be automated. It is a further advantage that the computer-implemented method can make complex modelling of eyes more accessible to a wider range of professionals and researchers, as it merely requires some ocular biometry parameters as input without further manual interventions by a qualified expert.

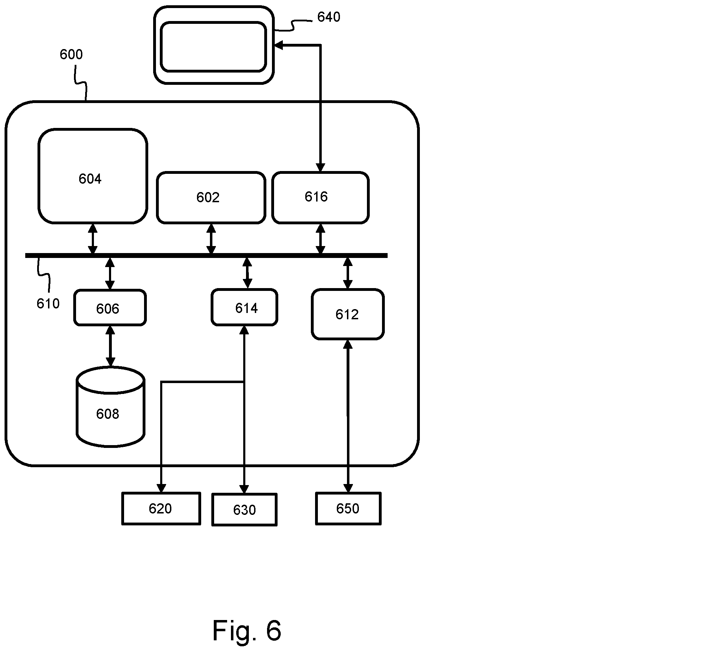

Fig. 6 shows a suitable computing system 600 enabling to implement embodiments of the above-described computer-implemented method according to the invention. Computing system 600 may in general be formed as a suitable general-purpose computer and comprise a bus 610, a processor 602, a local memory 604, one or more optional input interfaces 614, one or more optional output interfaces 616, a communication interface 612, a storage element interface 606, and one or more storage elements 608. Bus 610 may comprise one or more conductors that permit communication among the components of the computing system 600. Processor 602 may include any type of conventional processor or microprocessor that interprets and executes programming instructions. Local memory 604 may include a random-access memory (RAM) or another type of dynamic storage device that stores information and instructions for execution by processor 602 and/or a read only memory (ROM) or another type of static storage device that stores static information and instructions for use by processor 602. Input interface 614 may comprise one or more conventional mechanisms that permit an operator or user to input information to the computing device 600, such as a keyboard 620, a mouse 630, a pen, voice recognition and/or biometric mechanisms, a camera, etc. Output interface 616 may comprise one or more conventional mechanisms that output information to the operator or user, such as a display 640, etc. Communication interface 612 may comprise any transceiver-like mechanism such as for example one or more Ethernet interfaces that enables computing system 600 to communicate with other devices and/or systems 650. The communication interface 612 of computing system 600 may be connected to such another computing system by means of a local area network (LAN) or a wide area network (WAN) such as for example the internet. Storage element interface 606 may comprise a storage interface such as for example a Serial Advanced Technology Attachment (SATA) interface or a Small Computer System Interface (SCSI) for connecting bus 610 to one or more storage elements 608, such as one or more local disks, for example SATA disk drives, and control the reading and writing of data to and/or from these storage elements 608. Although the storage element(s) 608 above is/are described as a local disk, in general any other suitable computer-readable media such as a removable magnetic disk, optical storage media such as a CD or DVD, -ROM disk, solid state drives, flash memory cards, etc. could be used.

Although the present invention has been illustrated by reference to specific embodiments, it will be apparent to those skilled in the art that the invention is not limited to the details of the foregoing illustrative embodiments, and that the present invention may be embodied with various changes and modifications without departing from the scope thereof. The present embodiments are therefore to be considered in all respects as illustrative and not restrictive, the scope of the invention being indicated by the appended claims rather than by the foregoing description, and all changes which come within the meaning and range of equivalency of the claims are therefore intended to be embraced therein. In other words, it is contemplated to cover any and all modifications, variations or equivalents that fall within the scope of the basic underlying principles and whose essential attributes are claimed in this patent application. It will furthermore be understood by the reader of this patent application that the words "comprising" or "comprise" do not exclude other elements or steps, that the words "a" or "an" do not exclude a plurality, and that a single element, such as a computer system, a processor, or another integrated unit may fulfil the functions of several means recited in the claims. Any reference signs in the claims shall not be construed as limiting the respective claims concerned. The terms "first", "second", third", "a", "b", "c", and the like, when used in the description or in the claims are introduced to distinguish between similar elements or steps and are not necessarily describing a sequential or chronological order. Similarly, the terms "top", "bottom", "over", "under", and the like are introduced for descriptive purposes and not necessarily to denote relative positions. It is to be understood that the terms so used are interchangeable under appropriate circumstances and embodiments of the invention are capable of operating according to the present invention in other sequences, or in orientations different from the one(s) described or illustrated above.

## Claims

1. A computer-implemented method for generating an opto-mechanical model of a camera-type eye from ocular biometry parameters that define the anatomy of the camera-type eye; wherein the ocular biometry parameters comprise at least a pupil size (224) and an iris thickness (223); the computer-implemented method comprising (210):
- obtaining a posterior corneal surface (211), an inner scleral surface (212), and a crystalline lens surface (240);
- obtaining a predetermined iris shape (220) indicative for a generic shape of the iris in a general population, and a predetermined ciliary body shape (230) indicative for a generic shape of the ciliary body in the general population;
- anchoring (201) one or more data points (221) of an outer edge of the iris shape (220) to one or more reference points of the posterior corneal surface;
- transforming (202) the iris shape (220) as to respect the pupil size (224) and the iris thickness (223);
- anchoring (203) a first portion of the outer edge (250) of the ciliary body shape to a portion of an outer edge (225) of the iris shape;
- transforming (204) the ciliary body shape (230) such that a second portion of an outer edge (231) of the ciliary body shape coincides with a portion of the inner scleral surface (212); and
- generating (205) one or more polygons (261, 262) indicative for respective zonular fibres, wherein the respective one or more polygons are defined by at least two data points located on the crystalline lens surface (240) and at least two data points located on an inner edge (232) of the ciliary body shape.

2. The computer-implemented method according to claim 1, wherein anchoring (201) the one or more data points (221) of the outer edge of the iris shape to the one or more reference points of the posterior corneal surface comprises positioning (301) the iris shape (220) such that the one or more data points (221) coincide with the one or more reference points (302) of the posterior corneal surface (211).

3. The computer-implemented method according to any of the preceding claims, wherein the one or more anchored data points (221) are the data points on the outer edge of the iris shape (220) that are anatomically located at the greatest distance from a rotational axis (213) along a longitudinal axis (310) and anatomically located at the smallest distance to an apex (303) of the posterior corneal surface (211) along the rotational axis (213).

4. The computer-implemented method according to any of the preceding claims, wherein the one or more reference points (302) of the posterior corneal surface (211) are data points where the posterior corneal surface and the inner scleral surface (212) intersect.

5. The computer-implemented method according to any of the preceding claims, wherein anchoring (203) the first portion of the outer edge (250) of the ciliary body shape comprises positioning (401) the ciliary body shape such that the first portion of the outer edge (250) coincides with the portion of the outer edge (225) of the iris shape.

6. The computer-implemented method according to any of the preceding claims, wherein the first portion of the outer edge (250) of the ciliary body shape (230) and the portion of the outer edge (225) of the iris shape (220) coincide with the one or more reference points (302) of the posterior corneal surface (211).

7. The computer-implemented method according to any of the preceding claims, wherein the portion of the inner scleral surface (212) according to which the ciliary body shape is transformed (204) is the portion (402) of the inner scleral surface adjacent to the posterior corneal surface (211).

8. The computer-implemented method according to any of the preceding claims, wherein the ocular biometry parameters further comprise a length of a ciliary body (234) along a rotational axis (213) of the eye; and wherein transforming (204) the ciliary body shape (230) further comprises transforming the ciliary body shape as to respect the length (234) of the ciliary body along the rotational axis.

9. The computer-implemented method according to any of the preceding claims, wherein transforming (204) the ciliary body shape (230) further comprises transforming the ciliary body shape as to align a ciliary body apex (541) with a crystalline lens equator (542) along a longitudinal axis (310).

10. The computer-implemented method according to any of the preceding claims, wherein the at least two data points (501, 502, 511, 512) of the respective one or more polygons (261, 262) located on the crystalline lens surface (240) are located on the anterior lens surface (241) or posterior lens surface (242) at respective predetermined distances (521) from a crystalline lens equator (542) along a longitudinal axis (310).

11. The computer-implemented method according to any of the preceding claims, wherein the at least two data points (503, 504, 513, 514) of the respective one or more polygons (261, 262) located on the inner edge (232) of the ciliary body shape are located at respective predetermined distances (531) from a ciliary body apex (541) along a rotational axis (213).

12. The computer-implemented method according to any of the preceding claims, wherein the computer-implemented method is performed in a hemi-sagittal cross-sectional plane and further comprises rotating the shapes (220, 230, 261, 262) and surfaces (211, 212, 240) within the hemi-sagittal cross-sectional plane (210) around a rotational axis (213) to generate a three-dimensional, 3D, model of the eye.

13. A data-processing unit configured to perform the computer-implemented method according to any one of claims 1 to 12.

14. A computer program comprising instructions which, when the computer program is executed by a computer, cause the computer to perform the computer-implemented method according to any one of claims 1 to 12.

15. A computer-readable medium comprising instructions which, when executed by a computer, cause the computer to perform the computer-implemented method according to any of claims 1 to 12.
